# EUROPÄISCHE PATENTANMELDUNG

(11) **EP 3 922 277 A1**
(43) Veröffentlichungstag der Anmeldung: **15.12.2021**
(21) Anmeldenummer: 20179592.9
(22) Anmeldetag: 11.06.2020
(51) Int. Cl.: A61L 9/20

(54) **SYSTEM MIT WANDARTIGEN STRAHLUNGSFELDERN ZUR VERHINDERUNG ODER MINIMIERUNG DER VERBREITUNG VON VIREN IN RAUMLUFT**

(71) Anmelder: Smart United Holding GmbH, 82031 Grünwald (DE)
(72) Erfinder: Prohaska, Reiner, 82031 Grünwald (DE)
(74) Vertreter: Beder, Jens

(57) **Zusammenfassung**

Die Erfindung geht aus von einem System um eine Verbreitung von Viren zu verhindern oder zu minimieren und zur Verhinderung oder Minimierung der Verbreitung von Viren in Raumluft mit einer oder mehreren Strahlungsquellen (10) in einem Raum, die die Räume durch sogenannte UV-C Lichtwände den Raum in kleinere Segmente unterteilt, einer Sensoranordnung zum Detektieren einer Bewegung oder einer Anwesenheit einer oder mehrerer Personen (P) in dem Raum und einer Steuerung (16), die dazu ausgelegt ist, die eine oder mehreren Strahlungsquellen (10) abhängig von wenigstens der Anwesenheit der Person (P) ein- oder auszuschalten.

Es wird vorgeschlagen, dass die eine oder die mehreren Strahlungsquellen (10) dazu ausgelegt sind, jeweils ein wandartiges Strahlungsfeld (10b) zu erzeugen, welches als UV-C-Wand wirkt, dadurch den Raum oder die Räume in kleinere Raumsegmente aufzuteilen die eine Verbreitung von Viren verhindert oder minimiert, da die Viren durch das UV-C Licht deaktiviert werden, und die Steuerung (16) dazu ausgelegt ist, die betreffende Strahlungsquelle (10) abzuschalten, wenn die aus den von der Sensoranordnung detektierten Bewegungsdaten es wahrscheinlich erscheinen lassen, dass eine der Personen (P) das betreffende Strahlungsfeld (10b) durchqueren möchte.

## Beschreibung

Die Erfindung betrifft ein System mit wandartigen Strahlungsfeldern zur Desinfektion von Raumluft und um die Verbreitung von Viren in Gebäuden zu verhindern/minimieren.

Nicht erst seit der weltweiten Corona-Pandemie ist die gesundheitliche Bedeutung Hygienemaßnahmen in Räumen bekannt, insbesondere dort, wo eine Vielzahl von Personen zusammenarbeitet und/oder ein- und ausgeht.

Dabei wurden bereits verschiedene Systeme zur Desinfektion von Raumluft mit UV-Strahlung vorgeschlagen.

Zweckmäßige keimtötende Strahlung ist UV-Strahlung. Eine geeignete Strahlungsquelle ist daher eine UV-Lampe, die allgemein UV-Strahlung abgibt, die etwa im Wellenlängenbereich von 100 bis 400 nm liegt. Innerhalb der UV-Strahlung nimmt die keimtötende Wirkung von UV-A über UV-B bis UV-C mit kürzerer Wellenlänge zu. Besonders geeignet ist daher eine UV-C-Lampe nach den Ansprüchen 2 und 3, die speziell UV-C-Strahlung abgibt, die etwa im Wellenlängenbereich von 100 bis 280 nm liegt. Bevorzugt ist der Wellenlängenbereich etwa von 200 bis 280 nm, da Luft in diesem Bereich im Wesentlichen transparent für die Strahlung ist. Bekannte Strahlungsquellen dieser Art sind Quecksilberdampf-Lampen oder Leuchtdioden oder Laserdioden zur Abstrahlung entsprechenden UV-Lichts.

Keimtötende UV-C Strahlung kann schädlich für die menschlichen Augen und Haut sein. Maßnahmen, um daher Personen vor der Exposition mit der keimtötenden UV-C Strahlung zu schützen, können einen Spiegel, eine Blende und/oder einen Schirm zum Bündeln, Richten und Begrenzen der Strahlung umfassen. Vorzugsweise umfassen sie einen Sensor zur Erfassung der Anwesenheit einer Person im räumlichen Bereich, in dem die Strahlung wirken kann, insbesondere zur Erfassung der Anwesenheit einer Person im Bereich unmittelbar vor oder neben der Strahlungsquelle. Ein Schalter ist mit dem Sensor und der Strahlungsquelle gekoppelt und schaltet diese ab, wenn der Sensor die Person erfasst.

In dem Paper Welch, D., Buonanno, M., Grilj, V. et al. "Far-UV-C light: A new tool to control the spread of airborne-mediated microbial diseases" Sci Rep 8, 2752 (2018); https://doi.org/10.1038/s41598-018-21058-w wird allerdings beschrieben dass sehr kurzwelliges UV-C-Licht (207-222 nm), auch als Far-UV-C-Licht bezeichnet, effizient Bakterien inaktiviert, ohne die exponierte Haut von Säugetieren zu schädigen. Der Grund dafür ist, dass Far-UV-C-Licht aufgrund seiner starken Absorption in biologischen Materialien die äußeren (nicht lebenden) Schichten der menschlichen Haut oder des Auges nicht durchdringen kann. Da Bakterien und Viren jedoch Dimensionen haben von einem Mikrometer oder weniger habe, kann Far-UV-C-Licht in sie eindringen und inaktivieren. Es wird gezeigt, dass Far-UV-C aerosolisierte Viren in der Luft effizient inaktiviert, wobei eine sehr geringe Dosis von 2 mJ/cm² von 222-nm-Licht mehr als 95% eines aerosolisierten H1N1-Grip-pevirus inaktiviert.

Geeignete Sensoren zum Erfassen der Anwesenheit von Personen sind Bewegungsmelder wie beispielsweise Ultraschall- oder Radarsensoren, die den Dopplereffekt bei Reflektion der von ihnen abgegebenen Ultraschall- oder Radarstrahlung an einer sich bewegenden Person nutzen, oder passive pyroelektrische IR-Sensoren (PIR-Sensoren), die die von einer sich bewegenden Person verursachten Änderungen der Wärmestrahlung in der Umgebung des Möbels erfassen. Geeignet sind auch Näherungssensoren wie beispielsweise kapazitive, optische, Ultraschall- oder Radarsensoren, die eine Person in der Nähe unabhängig von ihrer Bewegung erfassen können.

Aus der WO 2016049143 A1 ist beispielsweise ein System zum Dekontaminieren von Nasszellen in Krankenhäusern bekannt. In der Nasszelle ist eine UV-C-Lichtquelle angeordnet, die abgeschaltet wird, sobald eine Person den Raum betritt.

Aus der US 9,550,006 B2 ist ein System mit UV-Strahlungsquellen zur Installation in Passagierkabinen von Flugzeugen bekannt. Ein Sicherheitssystem aktiviert oder deaktiviert die Strahlungsquellen, wenn ein Passagier oder Crew-Mitglied die Passagierkabine betritt.

Aus der US 9, 095, 633 B1 ist ein mobiles System zur Dekontamination von Krankenzimmern bekannt. Dieses System wird in dem Krankenzimmer aufgestellt und über einen Zeitschalter gestartet, wenn alle Personen den Raum verlassen haben.

Aus der WO 2015 054389 A2 sind UV-undurchlässige Strahlenschutzvorhänge bekannt, mit denen bestimmte zu desinfizierende Bereiche eines Raums (z.B. ein Bett eines Mehrbett-Krankenzimmers) abgetrennt werden können, um eine Desinfektion des abgetrennten Bereichs mit UV-Strahlungsquellen zu ermöglichen, während sich Personen in anderen Bereichen des Raums aufhalten können. Aus der WO 2014 100493 A1 ist ein ähnliches System zum gleichen Zweck mit Stellwänden bekannt, an deren Innenseite UV-Strahlungsquellen angeordnet sind.

Eine mobile UV-Strahlungsquelle, die in zu desinfizierenden Räumen oder mit UV-Schutz abgetrennten Raumbereichen aufgestellt werden kann, ist beispielsweise aus der WO 2012142427 A1 oder der US 6,656,424 B1 bekannt.

Ein Nachteil der oben genannten Systeme ist, dass der zu dekontaminierende Raum entweder vollständig menschenleer sein muss oder ein aufwändiger Aufbau von Strahlenschutzwänden oder Vorhängen erforderlich ist. In Räumen mit häufigem und unvorhersehbarem Publikumsverkehr ist dies nicht realisierbar.

Zur kontinuierlichen Desinfektion von Raumluft in Räumen, die dauerhaft belegt sind, beispielswiese Wartezimmern von Arztpraxen, ist es bekannt, UV-C-Strahlungsquellen innerhalb von Gehäusen von Lüftern, Klimaanlagen oder Ventilatoren anzuordnen. Aus der US 2009 004046 A1 ist beispielsweise eine derartige Vorrichtung zur Deckenmontage bekannt. Ein Nachteil dieser im Umluftverfahren arbeitenden Systeme ist, dass eine Diffusion von eventuell infektiösen Aerosolen zwischen Personen, die sich in dem betreffenden Raum befinden, möglich bleibt und die Aerosole sich durch den Luftzug der Umluft unter Umständen sogar noch schneller ausbreiten, als dies ohne die Umluft-System der Fall wäre.

Der Erfindung liegt die Aufgabe zugrunde, ein System mit wandartigen Strahlungsfeldern zur Verhinderung oder Minimierung der Verbreitung von Viren in Raumluft bereitzustellen, die eine Übertragung von Keimen zwischen Personen wirkungsvoll verhindert, ohne die Bewegungsfreiheit der Personen zu beeinträchtigen.

Die Aufgabe wird gelöst durch ein System mit wandartigen Strahlungsfeldern zur Verhinderung oder Minimierung der Verbreitung von Viren in Raumluft mit den Merkmalen des Anspruchs 1. Vorteilhafte Ausgestaltungen der Erfindung ergeben sich aus den Unteransprüchen.

Die Erfindung betrifft ein System zur Verhinderung oder Minimierung der Verbreitung von Viren in Raumluft mit einer oder mehreren Strahlungsquellen in einem Raum oder mehreren Räumen, insbesondere mit einer Sensoranordnung zum Detektieren einer Bewegung oder einer Anwesenheit einer oder mehrerer Personen in dem Raum und einer Steuerung, die dazu ausgelegt ist, die eine oder mehreren Strahlungsquellen abhängig von wenigstens der Anwesenheit der Person ein- oder auszuschalten.

Es wird vorgeschlagen, dass die eine oder die mehreren Strahlungsquellen dazu ausgelegt sind, durch gebündeltes UV-C-Licht jeweils ein wandartiges Strahlungsfeld zu erzeugen, welches als UV-C-Wand wirkt, dadurch den Raum oder die Räume in kleinere Raumsegmente aufzuteilen die eine Verbreitung von Viren verhindert oder minimiert, da die Viren durch das UV-C Licht deaktiviert werden. In Varianten mit einer Sensoranordnung und einer ist die Steuerung vorteilhaft dazu ausgelegt, die betreffende Strahlungsquelle abzuschalten, wenn die aus den von der Sensoranordnung detektierten Bewegungsdaten zeigen, dass eine der Personen sich dem betreffenden Strahlungsfeld nähert. Insbesondere wenn Far-UV-C-Strahlung verwendet wird, kann aus den oben beschriebenen Gründen auch auf die Sensoranordnung und das Abschalten verzichtet werden, da gesundheitliche Gefahren nicht zu befürchten sind.

Die wandartigen Strahlungsfelder, die insbesondere UV-C-Strahlung mit hoher Intensität enthalten, bilden Diffusionsbarrieren für Keime. Die Intensität und Wellenlänge des Strahlungsfelds ist so abgestimmt, dass Keime oder Viren, die in Aerosolen oder Tröpfchen enthalten sein können, beim Durchqueren des wandartigen Strahlungsfelds abgetötet werden. Die Ansteckungswahrscheinlichkeit kann so für Personen, die sich in durch ein derartiges Strahlungsfeld voneinander getrennten Raumsegmenten aufhalten, stark reduziert werden. Auch wenn die Viren nicht vollständig abgetötet werden, kann eine Wirkung erzielt werden, die derjenigen von Mund-Nasenschutzmasken oder "Social-Distancing" - Maßnahmen entspricht oder überlegen ist.

Ein Algorithmus, der eine Wahrscheinlichkeit für ein baldiges Durchqueren des Strahlungsfelds berechnet, kann neben der Position der Person, d.h. der Nähe der Person zum Strahlungsfeld, auch die Bewegungsrichtung und Bewegungsgeschwindigkeit der Person berücksichtigen sowie gewisse Randbedingungen des Raums. Solche Randbedingungen können beispielsweise durch Möbel definiert sein, deren Standorte in der Steuerung hinterlegt sind. Unter normalen Umständen wird es sehr unwahrscheinlich sein, dass eine Person über einen Tisch oder einen Raumteiler klettert oder springt.

Als "wandartiges" Strahlungsfeld soll ein insbesondere im Wesentlichen horizontal verlaufendes Strahlungsfeld bezeichnet werden, das im Wesentlichen eine zweidimensionale Fläche bildet, also eine Dicke hat, die im wenigstens eine Größenordnung kleiner ist als ihre Länge und ihre Breite. Das wandartige Strahlungsfeld kann insbesondere auch aus mehreren, eng nebeneinander liegenden, parallel orientierten Strahlen bestehen, beispielsweise Laserstrahlen.

Die Erfindung ist auf verschiedene Räume anwendbar, in denen sich Menschen aufhalten, z.B. Großraumbüros, Klassenzimmer in Schulen, Mehrbett-Krankenzimmer, Restaurants oder Arbeitsplätze in der Industrie.

Wegen der oben im Zusammenhang mit dem Paper von Welch et. Al. diskutierten Vorteile sind Ausführungsformen besonders vorteilhaft, in denen die Viren deaktivierende UV-C-Strahlung eine Far-UV-C Strahlung mit einer Wellenlänge im Bereich von 200 - 222 nm, insbesondere 207 - 222 nm ist. Wegen der technisch ausgereifteren Strahlungsquellen und aus Kostengründen kann in bestimmten Anwendungsgebieten auch ein Wellenlängenbereich von 223 - 280 nm vorteilhaft sein.

Ferner wird vorgeschlagen, dass die eine oder die mehreren Strahlungsquellen als Lichtleisten zur Decken- oder Wandmontage ausgestaltet sind. Jede der Strahlungsquellen kann mit einem oder mehreren UV-C-Strahlern, beispielsweise LEDs oder Laserdioden, ausgestattet sein oder eine stärkere UV-Lichtquelle wie eine Quecksilberdampflampe oder einen gepumpten Laser umfassen deren Licht dann durch eine geeignete optische Anordnung fächerartig aufgesplittet werden kann, um die gewünschte wandartige Form zu erzeugen. Durch die Ausgestaltung als montierbare Lichtleisten ist ein Flexibler Einsatz, auch beim Nachrüsten von Räumen, möglich.

Wenn das System freibewegliche Ständer zum Halten einer oder mehrerer Strahlungsquellen umfasst, ist das System auch in Bereichen einsetzbar, in denen die räumlichen Gegebenheiten eine Wand- oder Deckenmontage nicht erlauben.

In einer weiteren Ausgestaltung der Erfindung sind die eine oder die mehreren Strahlungsquellen jeweils zur Erzeugung mehrerer, parallel verlaufender Strahlungsfelder ausgelegt, so dass eine Doppelwand oder Mehrfachwand erzeugt wird. Dadurch kann die Schutzwirkung weiter verbessert werden.

Ferner wird vorgeschlagen, dass die eine oder die mehreren Strahlungsquellen zur Anordnung entlang von Begrenzungen von Raumsegmenten ausgelegt sind, wobei die Steuerung dazu ausgelegt ist, die betreffenden Strahlungsquellen zu aktivieren, wenn eine oder mehrere Personen sich in dem betreffenden Raumsegment aufhält und wenigstens eine der Strahlungsquellen zu deaktivieren, wenn eine Person das Raumsegment betritt oder verlässt.

Ferner wird vorgeschlagen, dass die Raumsegmente ein regelmäßiges Raster bilden. Dadurch können großflächige Räume flexibel abgedeckt werden.

In einer weiteren Ausgestaltung der Erfindung sind in den Raumsegmenten weitere Strahlungsquellen mit desinfizierender bzw. Viren deaktivierender Wirkung angeordnet. Die Steuerung kann dann dazu ausgelegt sein, die weiteren Strahlungsquellen zu aktivieren, wenn sich keine Person in dem Raumsegment befindet. Dadurch können Oberflächen, Computer, Stühle etc. wirkungsvoll desinfiziert werden, währen keine Person sich in dem Raumsegment aufhält.

Ferner wird vorgeschlagen, dass die Sensoranordnung eine 3D-Kamera bzw. TOF-Kamera und/oder eine oder mehrere CCD-Kameras umfasst, um die dreidimensionale Position und Pose der Personen in dem betreffenden Raumsegment detektieren zu können und auswerten zu können.

Ein weiterer Aspekt der Erfindung betrifft ein Verfahren zur Verhinderung oder Minimierung der Verbreitung von Viren in Raumluft mit einer oder mehreren Strahlungsquellen in einem Raum, optional umfassend das Detektieren einer Bewegung oder einer Anwesenheit einer oder mehrerer Personen in dem Raum und das automatische Ein- oder Ausschalten einer oder mehrerer Strahlungsquellen abhängig von wenigstens der Anwesenheit der Person.

Es wird vorgeschlagen, dass die eine oder die mehreren Strahlungsquellen dazu ausgelegt sind, jeweils ein wandartiges Strahlungsfeld zu erzeugen, , welches als UV-C-Wand wirkt, dadurch den Raum oder die Räume in kleinere Raumsegmente aufzuteilen die eine Verbreitung von Viren verhindert oder minimiert, da die Viren durch das UV-C Licht deaktiviert werden und das Verfahren das Abschalten der betreffenden Strahlungsquelle, wenn die aus den von der Sensoranordnung detektierten Bewegungsdaten es wahrscheinlich erscheinen lassen, dass eine der Personen das betreffende Strahlungsfeld durchqueren möchte, umfasst.

Die Erfindung betrifft zudem ein System um Verbreitung von Viren in Räumen zu verhindern oder zu minimieren und zur Desinfektion von Raumluft mit einer oder durch mehrere zusammen geschalteten Strahlungsquellen dadurch gekennzeichnet, dass die eine oder die mehrere zusammen geschalteten Strahlungsquellen durch gebündeltes UV-C Licht, sogenannte Lichtwände bilden und dadurch Räume in kleinere Segmente aufteilt die eine Verbreitung von Viren verhindert oder minimiert, da die Viren durch das UV-C Licht deaktiviert werden, in Kombination mit Bewegungsmeldern die einzelne UV-C Lichtwände ausschalten wenn sich eine Person nähert oder sich wieder einschalten wenn die Person sich entfernt und einem zusätzlichen UV-C Strahler der die einzelnen durch die eine oder mehrere UV-C Lichtwände entstanden Parzelle aufgeteilten Räume ausstrahlt und die Aerosole (in der Luft vorhandenen Viren) deaktiviert.

Weitere Merkmale und Vorteile ergeben sich aus der folgenden Figurenbeschreibung. Die gesamte Beschreibung, die Ansprüche und die Figuren offenbaren Merkmale der Erfindung in speziellen Ausführungsbeispielen und Kombinationen. Der Fachmann wird die Merkmale auch einzeln betrachten und zu weiteren Kombinationen oder Unterkombinationen zusammenfassen, um die Erfindung, wie sie in den Ansprüchen definiert ist, an seine Bedürfnisse oder an spezielle Anwendungsbereiche anzupassen.

Dabei zeigen:
Fig. 1 ein System zur Verhinderung oder Minimierung der Verbreitung von Viren in Raumluft nach einem ersten Ausführungsbeispiel der Erfindung;
Fig. 2a - 2c ein einzelnes Raumsegment des Systems aus Fig. 1 in drei verschiedenen Zuständen;
Fig. 3a und 3b eine schematische Schnittansicht einer Strahlungsquelle und eines wandartigen Strahlungsfelds gemäß zwei verschiedenen Ausführungsbeispielen der Erfindung;
Fig. 4 zeigt eine weitere Ausgestaltung der Erfindung mit einem Ständer für die Strahlungsquellen eines erfindungsgemäßen Systems.

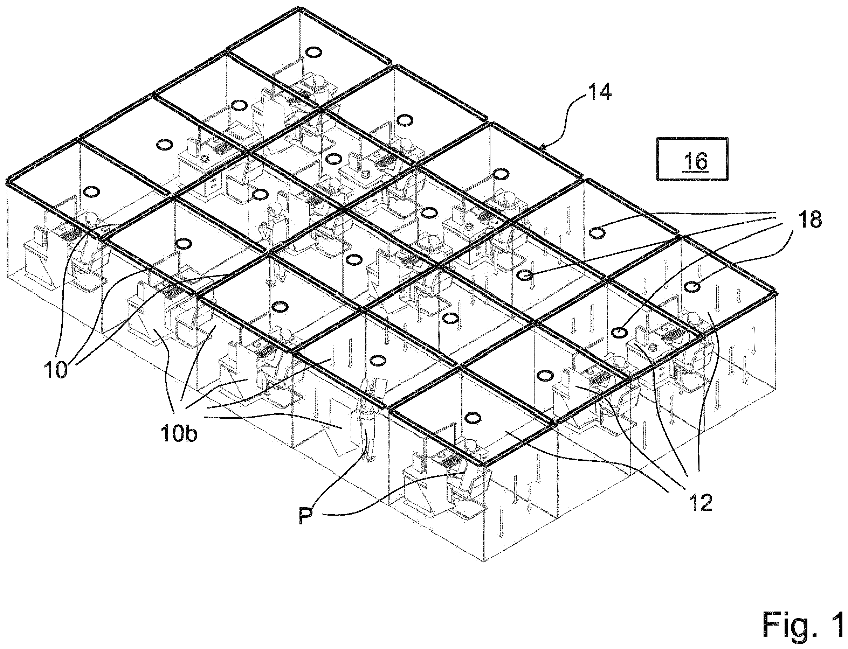

Figur 1 zeigt ein erstes Ausführungsbeispiel der Erfindung, und zwar ein System zur Verhinderung oder Minimierung der Verbreitung von Viren in Raumluft in einem Großraumbüro. Das Großraumbüro hat einen in quadratische Raumsegmente aufgeteilten Grundriss mit in Reihen angeordneten Arbeitsplätzen und Gängen. Jeder Arbeitsplatz ist mit einem Arbeitstisch einem Stuhl und Ablagen ausgestattet. Die Erfindung ist aber auch auf andere Räume anwendbar, beispielsweise solche mit unterschiedlich großen Arbeitsplätzen oder Büros mit Open-Space-Konzept.

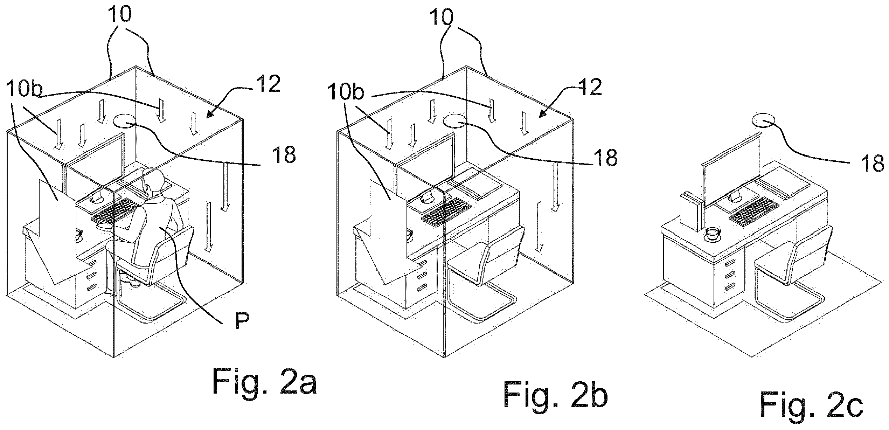

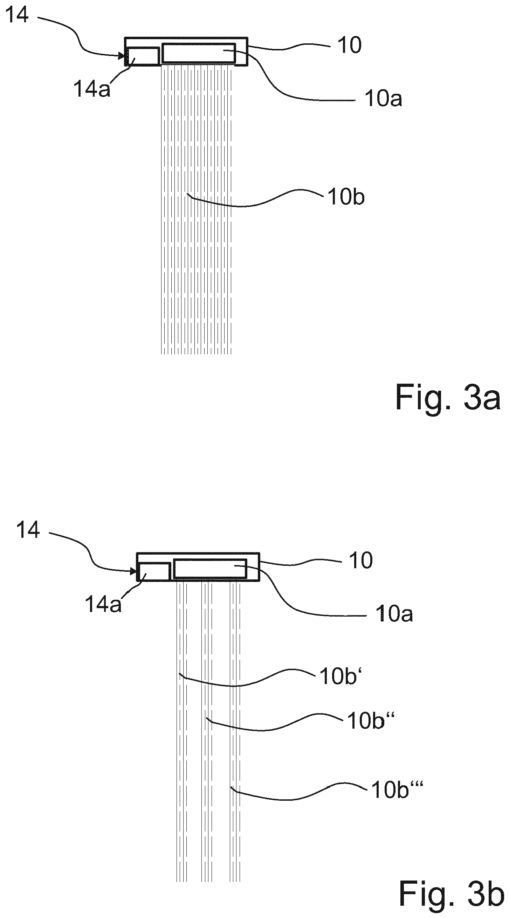

An der Decke des Raums ist eine gitterartige Anordnung von Strahlungsquellen 10 angebracht. Jede der Strahlungsquellen 10 ist eine Lichtleiste mit einem oder mehreren UV-C-Strahlern 10a (Fig. 3a, 3b), beispielsweise einer Quecksilberdampf-Entladungslampe, LEDs oder Laserdioden, und erzeugt jeweils ein wandartiges Strahlungsfeld 10b. Das Strahlungsfeld 10b kann insbesondere kurzwellige Far-UV-C-Strahlung mit Wellenlängen im Bereich von 207 - 222 nm enthalten. Durch geeignete Filter können schädliche Wellenlängen herausgefiltert werden. Zur Erzeugung der Far-UV-C-Strahlung kommen insbesondere Excimerlampen mit einer Kr-Cl-Gasmischung in Betracht. Im Folgenden werden die wandartigen Strahlungsfelder 10b der Einfachheit halber auch als UV-C-Wand bezeichnet. In Fig. 1, 2a und 2b sind die eigentlich unsichtbaren UV-C-Wände 10b als vertikal nach unten gerichtete, weiße Pfeile dargestellt.

Zur Erzeugung eines wandartigen Strahlungsfelds 10b kann Strahlung optisch oder durch Spaltblenden zu parallelen Strahlen gebündelt bzw. kollimiert werden. Alternativ oder ergänzend dazu kann das Strahlungsfeld 10b durch eine Anordnung von parallelen Laserstrahlen mit lateral überlappenden Strahlungsprofilen erzeugt werden. Eine weitere Alternative wäre ein oder mehrere schnell in einer Fläche hin- und herbewegte bzw. gescannte Laserstrahlen - ähnlich wie bei Barcode-Scannern - wobei die Scangeschwindigkeit und der Strahldurchmesser so aufeinander abgestimmt sind, dass jedes durch die UV-C-Wand 10b hindurch diffundierende Aerosol einer hinreichenden Strahlungsdosis ausgesetzt ist.

Die Raumsegmente 12 sind jeweils durch UV-C-Wände 10b voneinander abgegrenzt. In dem in Fig. 1 dargestellten Ausführungsbeispiel ist jedes Raumsegment 12 von vier UV-C-Wänden 10b begrenzt.

Auch wenn in Fig. 1 solche Sonderfälle nicht dargestellt sind, bilden vorhandene solide Wände des Raums bilden Begrenzungen der Raumsegmente 12, so dass Raumsegmente 12 am Rand oder in Ecken des Raums neben den vorhandenen soliden Wänden nur durch drei bzw. zwei weitere UV-C-Wände 10b abgegrenzt werden müssen. Es sind auch Ausgestaltungen der Erfindung denkbar, in denen ein mit soliden Wänden abgegrenzter Raum, z.B. ein Einzelbüro, ein akustisch abgeschirmter Besprechungsbereich etc. ein Raumsegment 12 bilden, welches Aerosole mit anderen Raumsegmenten 12 nur durch eine Türöffnung oder einen Zugang austauschen kann. In diesem Fall ist es ausreichend, nur die betreffende Türöffnung bzw. den Zugang mit einer UV-C-Wand 10b von den restlichen Raumsegmenten 12 abzuschirmen.

Strukturen wie halbhohe Wände, Raumteiler oder dergleichen können durch eine UV-C-Wand 10b bis zur Decke fortgesetzt bzw. erweitert werden. In diesem Fall könnten die Strahlungsquellen 10 auch auf der Oberseite der betreffenden Struktur montiert werden und nach oben zur Decke hin strahlen.

In den Lichtleisten 10 sind zudem Sensoren 14a (Fig. 3a, 3b) einer Sensoranordnung 14 zum Detektieren einer Bewegung oder einer Anwesenheit einer oder mehrerer Personen P in dem Raum angeordnet.

Eine zentrale Steuerung 16 ist durch eine geeignete Software dazu ausgelegt, die eine oder mehreren Strahlungsquellen 10 abhängig von wenigstens der Anwesenheit der Person P ein- oder auszuschalten, wie dies im Folgenden detaillierter beschrieben wird. Die Steuerung 16 kommuniziert dazu über Signalleitungen oder drahtlos, beispielsweise über WLAN, mit den Strahlungsquellen 10.

Die Steuerung 16 wertet die Positions- und Bewegungsdaten der Personen P aus und berechnet Wahrscheinlichkeiten für verschiedene Wege bzw. Bewegungen der Personen P. Wenn eine Person P ruhig und in hinreichender Entfernung von allen UV-C-Wänden 10b an ihrem Arbeitsplatz sitzt, ist es unwahrscheinlich, dass sie im nächsten Sekundenbruchteil eine der UV-C-Wände 10b durchquert. Wenn die Person aber zügig durch einen Gang läuft, der durch mehrere UV-C-Wände 10b in Raumsegmente 12 aufgeteilt ist, ist der Zeitpunkt des Durchquerens der nächsten UV-C-Wand 10b gut vorhersehbar. Wegen der gesundheitlichen Risiken werden die Strahlungsquellen 10 bereits bei einer geringen Wahrscheinlichkeit abgeschaltet, wobei der Schwellenwert bei der Verwendung von Far-UV-C-Strahlen wegen der geringeren Risiken auf einen höheren Wert gesetzt werden kann als bei längerwelligen UV-Strahlungstypen.

Wenn anhand der von der Sensoranordnung 14 detektierten Bewegungsdaten eine hinreichende Wahrscheinlichkeit für das Durchqueren der UV-C-Wand 10b festgestellt wurde, schaltet die Steuerung 16 die betreffende Strahlungsquelle 10 ab.

Die Personen P können sich daher frei im Raum bewegen. Wenn die Person P dabei eine Grenzfläche zwischen zwei Raumsegmenten 12 durchquert, schaltet die Steuerung 16 die diese Grenzfläche bildende UV-C-Wand 10b ab und schaltet die UV-C-Wand 10b wieder ein, wenn sich die Person vollständig im zweiten Raumsegment 12 befindet.

Während sich eine oder mehrere Personen P sich in dem betreffenden Raumsegment 12 aufhält, bleiben in der Regel die betreffenden Strahlungsquellen 10 aktiv, so dass Viren und Bakterien in Tröpfchen oder Aerosolen beim Verlassen des Raumsegments 12 abgetötet werden. Dadurch werden Personen P, die sich in unterschiedlichen Raumsegmenten 12 befinden, voneinander abgeschirmt. Da die Strahlungsquellen 10 aktiv bleiben, während sich Personen P in den Raumsegmenten 12 aufhalten, können zur Vermeidung gesundheitlicher Schäden durch Streulicht Absorber-Leisten auf dem Boden angebracht sein, die das aus den Strahlungsquellen 10 einfallende UV-C-Licht absorbieren.

Nur wenn eine Person P das Raumsegment 12 durch eine UV-C-Wand 10b betreten oder verlassen möchte, wird die der entsprechenden UV-C-Wand 10b zugeordnete Strahlungsquelle 10 deaktiviert.

Mittig an der Decke sind in den Raumsegmenten 12 weitere Strahlungsquellen 18 mit Viren deaktivierender bzw. desinfiziernder Wirkung angebracht. Die Steuerung 16 ist dazu ausgelegt, die weiteren Strahlungsquellen 18 für ein vorgegebenes Zeitintervall zu aktivieren, wenn sich keine Person in dem Raumsegment 12 befindet. Auch diese Strahlungsquellen 18 werden abgeschaltet, wenn eine Person P das betreffende Raumsegment 12 betritt. Um für die Person P kenntlich zu machen, ob die Desinfektion des betreffenden Raumsegments 12 abgeschlossen ist, kann eine Leuchtdiode oder ein Ampelsystem vorgesehen sein. Es sind weitere Ausgestaltungen der Erfindung denkbar, in welchen die Sensoranordnung 14 Sensoren umfasst, die in die Strahlungsquellen 18 integriert sind. Die Strahlungsquellen 18 können in Deckenverkleidungskacheln, Lampen oder Lüftungsgitter integriert sein oder mit anderen Geräten, z.B. Rauchmeldern, in ein Gehäuse integriert sind.

Die Fig. 2a - 2c zeigen ein einzelnes Raumsegment 12 des Systems aus Fig. 1 in drei verschiedenen Zuständen.

In dem in Fig. 2a dargestellten Arbeits-Zustand arbeitet eine Person P in dem von vier UV-C-Wänden 10b abgegrenzten Raumsegment 12. Alle vier UV-C-Wände 10b sind eingeschaltet, so dass in Aerosolen enthaltene Keime beim Durchqueren der Grenzflächen zwischen benachbarten Raumsegmenten 12 inaktiviert werden.

In dem in Fig. 2b dargestellten Desinfektions-Zustand hat eine Person P in dem Raumsegment 12 gearbeitet und es verlassen. Beim Verlasse des Raumsegments 12 aufgrund der detektierten Bewegung der Person P eine der vier UV-C-Wände 10b abgeschaltet (nicht dargestellt). Alle vier UV-C-Wände 10b sind eingeschaltet, so dass keine aktiven Keime austreten können. Zusätzlich wird die mittig an der Decke angebrachte Strahlungsquelle 18 für einen vorgegebenen Zeitraum aktiviert, um auch die auf den Oberflächen des Arbeitsplatzes und innerhalb des Raumsegments 12 schwebenden Keime abzutöten.

In dem in Fig. 2c dargestellten Ruhe-Zustand ist die Desinfektion abgeschlossen und keine Person P hält sich in dem Raumsegment 12 auf. Zum Energiesparen werden alle vier UV-C-Wände 10b und auch die mittig an der Decke angebrachte Strahlungsquelle 10 abgeschaltet.

Fig. 3a zeigt eine schematische Schnittansicht einer Strahlungsquelle 10 und eines wandartigen Strahlungsfelds 10b nach dem ersten Ausführungsbeispiel der Erfindung. Das Strahlungsfeld 10b hat eine im Rahmen der optischen Möglichkeiten konstante Dicke von etwa 1 cm.

Wie oben beschrieben implementiert die Steuerung 16 ein Verfahren zur Verhinderung oder Minimierung der Verbreitung von Viren in Raumluft mit einer oder mehreren Strahlungsquellen 10 in einem Raum. Das Verfahren umfasst das Detektieren einer Bewegung oder einer Anwesenheit einer oder mehrerer Personen P in dem Raum und das automatische Ein- oder Ausschalten einer oder mehrerer Strahlungsquellen 10 abhängig von wenigstens der Anwesenheit der Person P.

Nach dem Verfahren wird die betreffende Strahlungsquelle 10 abgeschaltet, wenn die aus den von der Sensoranordnung 14 detektierten Bewegungsdaten es wahrscheinlich erscheinen lassen, dass eine der Personen P das betreffende Strahlungsfeld 10b durchqueren möchte.

Die Figur 3b zeigt ein weiteres Ausführungsbeispiel der Erfindung. Um Wiederholungen zu vermeiden, beschränkt sich die nachfolgende Beschreibung dieser weiteren Ausführungsbeispiele im Wesentlichen auf Unterschiede zu dem ersten Ausführungsbeispiel der Erfindung. Wegen der unveränderten Merkmale wird der Fachmann auf die Beschreibung zum ersten Ausführungsbeispiel verwiesen. Für gleich oder ähnlich wirkende Merkmale der weiteren Ausführungsbeispiele werden gleiche Bezugszeichen verwendet, um die Ähnlichkeiten zu betonen.

In dem in Figur 3b dargestellten Ausführungsbeispiel sind die Strahlungsquellen 10 jeweils zur Erzeugung mehrerer, parallel verlaufender Strahlungsfelder 10b' - 10b'" ausgelegt, die beispielsweise eine Dicke von 1 mm und einen Abstand von 1 mm haben können.

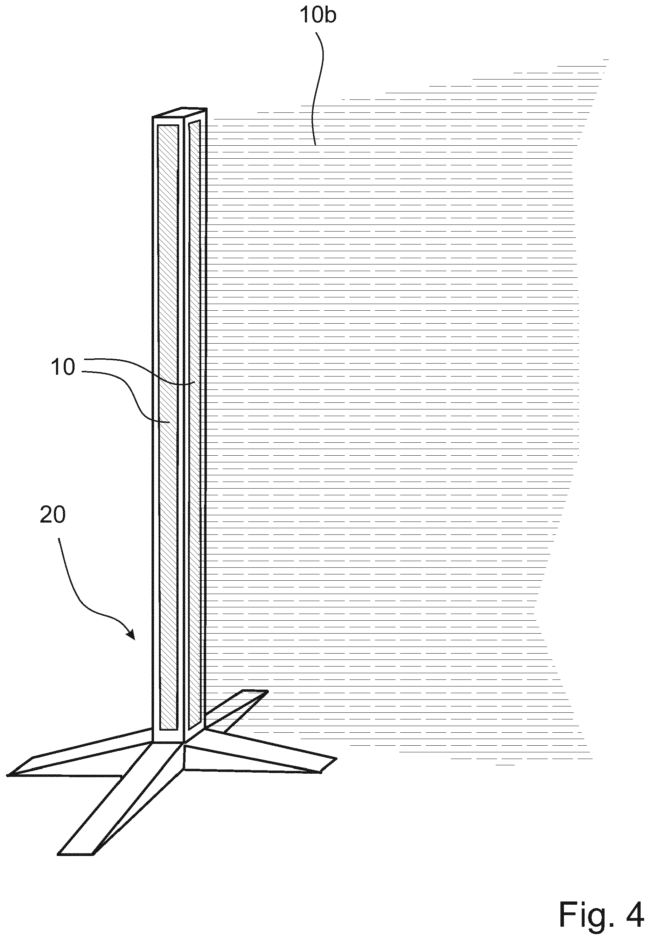

Fig. 4 zeigt eine weitere Ausgestaltung der Erfindung mit einem Ständer 20 für die Strahlungsquellen 10 eines erfindungsgemäßen Systems, die UV-C-Strahlung in einer horizontalen Richtung abstrahlen um so UV-C-Wände 10b zu bilden. Je nach Anwendungsbereich kann ein Ständer 20 mit einer, zwei, drei oder vier Strahlungsquellen 10 ausgestattet sind, die ausgehend von dem Ständer 20 bis zu vier in verschiedene Raumrichtungen ausstrahlende UV-C-Wände 10b erzeugen können. Das abgestrahlte UV-C-Licht kann von benachbarten Ständern oder zu diesem Zweck aufgestellten Absorber-Wänden oder Absorber-Ständern absorbiert werden.

In weiteren, nicht dargestellten Ausführungsformen der Erfindung können die Ständer vertikal nach unten strahlende Lichtleisten bzw. Strahlungsquellen halten. Ferner ist es denkbar, dass die Lichtleisten bzw. Strahlungsquellen auf dem Boden liegen und zur Decke hin strahlen.

## Patentansprüche

1. System zur Verhinderung oder Minimierung der Verbreitung von Viren in Raumluft mit einer oder mehreren Strahlungsquellen (10) in einem Raum oder mehreren Räumen,
**dadurch gekennzeichnet, dass**
die eine oder die mehreren Strahlungsquellen (10) dazu ausgelegt sind, durch gebündeltes UV-C Licht jeweils ein wandartiges Strahlungsfeld (10b) mit Viren deaktivierender UV-C-Strahlung zu erzeugen, welches als UV-C-Wand wirkt, dadurch den Raum oder die Räume in kleinere Raumsegmente aufzuteilen die eine Verbreitung von Viren verhindert oder minimiert, da die Viren durch das UV-C Licht deaktiviert werden.

2. System nach Anspruch 1,
**dadurch gekennzeichnet, dass**
ferner umfassend eine Sensoranordnung (14) zum Detektieren einer Bewegung oder einer Anwesenheit einer oder mehrerer Personen (P) in dem Raum und eine Steuerung (16), die dazu ausgelegt ist, die eine oder mehreren Strahlungsquellen (10) abhängig von wenigstens der Anwesenheit der Person (P) ein- oder auszuschalten, wobei die Steuerung (16) dazu ausgelegt ist, die betreffende Strahlungsquelle (10) abzuschalten, wenn die aus den von der Sensoranordnung (14) detektierten Bewegungsdaten zeigen, dass eine der Personen (P) sich dem betreffenden Strahlungsfeld (10b) nähert.

3. System nach Anspruch 1 oder 2,
**dadurch gekennzeichnet, dass**
die Viren deaktivierende UV-C-Strahlung, die zu einer UV-C-Wand gebündelt wird, eine Far-UV-C Strahlung mit einer Wellenlänge im Bereich von 200 - 222 nm, insbesondere 207 - 222 nm ist.

4. System nach Anspruch 1 oder 2,
**dadurch gekennzeichnet, dass**
die Viren deaktivierende UV-C-Strahlung, die zu einer UV-C-Wand gebündelt wird, eine UV-C Strahlung mit einer Wellenlänge im Bereich von 223 - 280 nm ist.

5. System nach einem der vorhergehenden Ansprüche,
**dadurch gekennzeichnet, dass**
die eine oder die mehreren Strahlungsquellen (10) als Lichtleisten zur Decken- oder Wandmontage ausgestaltet sind.

6. System nach einem der vorhergehenden Ansprüche,
**gekennzeichnet durch** freibewegliche Ständer (20) zum Halten einer oder mehrerer Strahlungsquellen (10).

7. System nach einem der vorhergehenden Ansprüche,
**dadurch gekennzeichnet, dass**
die eine oder die mehreren Strahlungsquellen (10) jeweils zur Erzeugung mehrerer, parallel verlaufender Strahlungsfelder (10b' - 10b'") ausgelegt sind.

8. System nach einem der vorhergehenden Ansprüche,
**dadurch gekennzeichnet, dass**
die eine oder die mehreren Strahlungsquellen (10) zur Anordnung entlang von Begrenzungen von Raumsegmenten (12) ausgelegt sind, wobei die Steuerung (16) dazu ausgelegt ist, die betreffenden Strahlungsquellen (10) zu aktivieren, wenn eine oder mehrere Personen (P) sich in dem betreffenden Raumsegment (12) aufhält und wenigstens eine der Strahlungsquellen (10) zu deaktivieren, wenn eine Person (P) das Raumsegment (12) betritt oder verlässt.

9. System nach Anspruch 8,
**dadurch gekennzeichnet, dass**
die Raumsegmente (12) ein regelmäßiges Raster bilden.

10. System nach einem der Ansprüche 8 oder 9,
**dadurch gekennzeichnet, dass**
innerhalb der Raumsegmenten (12) weitere Strahlungsquellen (18) mit Viren deaktivierender und/oder desinfizierender Wirkung angeordnet sind und die Steuerung (16) dazu ausgelegt ist, die weiteren Strahlungsquellen (10) zu aktivieren, wenn sich keine Person (P) in dem Raumsegment (12) befindet.

11. System nach einem der vorhergehenden Ansprüche,
**dadurch gekennzeichnet, dass**
die Sensoranordnung eine 3D-Kamera bzw. TOF-Kamera und/oder eine oder mehrere CCD-Kameras umfasst.

12. Verfahren zur Verhinderung oder Minimierung der Verbreitung von Viren in Raumluft mit einer oder mehreren Strahlungsquellen (10) in einem Raum,
**dadurch gekennzeichnet, dass**
das Verfahren das Erzeugen wenigstens eines wandartigen Strahlungsfelds (10b) durch gebündeltes UV-C-Licht mittels der einen oder den mehreren Strahlungsquellen (10) umfasst, wobei das Strahlungsfeld (10b) als UV-C-Wand wirkt, wobei dadurch der Raum oder die Räume in kleinere Raumsegmente aufgeteilt werden, wobei die UV-C-Wand eine Verbreitung von Viren verhindert oder minimiert, da die Viren durch das UV-C Licht deaktiviert werden.

13. Verfahren nach Anspruch 12, ferner umfassend das Detektieren einer Bewegung oder einer Anwesenheit einer oder mehrerer Personen (P) in dem Raum und das automatische Ein- oder Ausschalten einer oder mehrerer Strahlungsquellen (10) abhängig von wenigstens der Anwesenheit der Person (P), und
das Abschalten der betreffenden Strahlungsquelle (10), wenn die aus den von der Sensoranordnung detektierten Bewegungsdaten es wahrscheinlich erscheinen lassen, dass eine der Personen (P) das betreffende Strahlungsfeld (10b) durchqueren möchte.
